# EUROPEAN PATENT SPECIFICATION

(11) **EP 1 660 889 B1**
(45) Date of publication and mention of the grant of the patent: **11.05.2011**
(21) Application number: 04761638.8
(22) Date of filing: 16.08.2004
(51) Int. Cl.: G01N 33/66, G01N 33/74

(54) **DIAGNOSTIC COMPOSITION FOR DIABETES TYPE-2 AND IMPAIRED GLUCOSE TOLERANCE, AND METHODS OF USE**
DIAGNOSTISCHE ZUSAMMENSETZUNG FÜR TYP-2-DIABETES UND BEEINTRÄCHTIGTE GLUCOSETOLERANZ SOWIE VERWENDUNGSVERFAHREN
COMPOSITION DIAGNOSTIQUE POUR LE DIABETE DE TYPE 2 ET L'INTOLERANCE AU GLUCOSE, ET SES PROCEDES D'UTILISATION

(30) Priority: 13.08.2003 US 494549 P
(43) Date of publication of application: 31.05.2006
(73) Proprietor: Ceapro Inc., Edmonton, Alberta T6G 2E1 (CA)
(72) Inventor: REDMOND, Mark, J., Edmonton, Alberta T6G 1B3 (CA); SHAW, Diana, F., Edmonton, Alberta T5K 2M3 (CA)
(74) Representative: Harding, Charles Thomas
(86) International application number: PCT/CA2004/001474
(87) International publication number: WO 2005/017532

(56) References cited:
- WO-A-97/02050
- WO-A2-97/02050
- US-B1- 6 248 375
- KAY R M ET AL: "Diets rich in natural fibre improve carbohydrate tolerance in maturity-onset, non-insulin dependent diabetics." DIABETOLOGIA 1981, vol. 20, no. 1, 1981, pages 18-21, XP009100836 ISSN: 0012-186X
- BENGOA J M ET AL: "Glycaemic response and hydrogen production to blenderised and polymeric enteral nutrition formulas." CLINICAL NUTRITION (EDINBURGH, SCOTLAND) NOV 1986, vol. 5, no. 4, November 1986 (1986-11), pages 209-212, XP002482171 ISSN: 0261-5614
- LU Z X ET AL: "Arabinoxylan fiber, a byproduct of wheat flour processing, reduces the postprandial glucose response in normoglycemic subjects." THE AMERICAN JOURNAL OF CLINICAL NUTRITION MAY 2000, vol. 71, no. 5, May 2000 (2000-05), pages 1123-1128, XP002482172 ISSN: 0002-9165
- WOLEVER T M ET AL: "Variation of postprandial plasma glucose, palatability, and symptoms associated with a standardized mixed test meal versus 75 g oral glucose." DIABETES CARE MAR 1998, vol. 21, no. 3, March 1998 (1998-03), pages 336-340, XP002482173 ISSN: 0149-5992
- WOLEVER T M ET AL: "The glycemic index: methodology and clinical implications." THE AMERICAN JOURNAL OF CLINICAL NUTRITION NOV 1991, vol. 54, no. 5, November 1991 (1991-11), pages 846-854, XP002482174 ISSN: 0002-9165
- JENKINS ET AL.: 'Depression of the glycemic index by high levels of beta-glucan fibre in two functional foods tested in type 2 diabetes' EUR. J. CLIN. NUTR. vol. 56, no. 7, July 2002, pages 622 - 628, XP002476550
- KABIR ET AL.: 'Four-week low-glycemic index breakfast with a modest amount of soluble fibres in type 2 diabetes men' METABOLISM vol. 51, no. 7, July 2002, pages 819 - 826, XP008096968

## Description

### FIELD OF THE INVENTION

The present invention relates generally to products for use in the detection of metabolic disease. In particular, the invention relates to a dry, baked test meal useful in the screening and early diagnosis of impaired glucose tolerance and type-2 diabetes.

### BACKGROUND OF THE INVENTION

Disorders of carbohydrate metabolism traditionally include diabetes and impaired glucose tolerance. Diabetes is a well-recognized cause of significant morbidity and mortality. With the World Health Organization ("WHO") estimating 300 million diabetics globally by 2025, non-insulin dependent, type 2, diabetes ("type-2 diabetes") is a major public health concern. Type-2 diabetes is the most common diagnosis of patients entering dialysis programs in the United States, a major cause of vision loss and a major contributing factor in cardiac, peripheral, and cerebral vascular diseases.

The Da Qing IGT and Diabetes Study and Bayer AG STOP-NIDDM study have the objectives of determining the effects of diet, exercise and drug interventions in preventing type-2 diabetes in people with impaired glucose tolerance ("IGT"). Results to date suggest that early diagnosis and treatment of impaired glucose tolerance and type-2 diabetes to reduce hyperglycemia, may delay the onset and reduce complications, respectively, of type-2 diabetes.

A patient with impaired glucose tolerance displays an abnormal glucose tolerance in which the blood glucose levels are not high enough to be associated with the specific complications of diabetes. Impaired glucose tolerance is a major risk factor in the development of type-2 diabetes, peripheral vascular disease and cardiovascular disease. However, despite these facts, widespread screening for impaired glucose tolerance and symptomatic and/or undiagnosed type-2 diabetes has not been routinely conducted.

The fasting plasma glucose ("FPG") and the random plasma glucose ("RPG") tests are the most commonly utilized screening test methods for diabetes. The method for FPG requires an overnight fast, after which a blood sample is taken and blood glucose level determined. The RPG test comprises a blood glucose test regardless of the time since the last ingestion of food. The use of fasting or random glucose alone is considered to lack adequate specificity and sensitivity (Modan et al. (1994) Diabetes Care 17:436-439). Further, these tests cannot detect impaired glucose tolerance. However, since these are the most rapid, simple and cost effective tests, the fasting glucose and random glucose screens are the current methods of choice and recommended by the American Diabetic Association.

The accepted method of diagnosing diabetes, impaired glucose tolerance, type-2 diabetes, and hyperinsulinemia is to administer a 75-gram oral glucose tolerance test ("OGTT"). The main reason for the use of this test is that a postprandial blood glucose value is needed to make an accurate diagnosis. Glucose is used because it is easy to standardize the amount administered, it is easily stored, and its absorption is not influenced by other food factors such as protein or fat, cooking, and processing. However, the OGTT has drawbacks and is not commonly utilized as a clinical test. Thus, diabetes, impaired glucose tolerance, and hyperinsulinemia are not generally diagnosed early.

One of the drawbacks of the OGTT is the difficulty conducting the test, which requires at least an 8-hour fast and a timed blood sample 120 minutes after consuming 75 grams of liquid glucose. In addition, glucose is generally unpalatable and 75 grams is a large dose that may lead to nausea and other gastrointestinal side-effects. Moreover, the results of the OGT test are highly variable often leading to false positives and false negatives. Because of this variability, it is difficult to interpret the results of an OGTT.

Blood glucose can be tested either using venous whole blood or capillary whole blood samples, though the diagnostic levels will vary depending from where the blood is drawn. Blood glucose levels are significantly higher in capillary blood samples than they are in venous blood samples (Kuwa et al., Clin Chim Acta (2001) May 307 (1-2):187-92). The diagnosis of diabetes is established if fasting venous plasma glucose is at least 160 mg/dL (7.8 mmol/L) or plasma glucose is at least 200 mg/dL (11.1 mmol/L).

Near-normal glycemic control can prevent diabetic complications. However, early interest in mass screening for diabetes to facilitate early diagnosis and prevent debilitating or fatal complications was tempered by the undesirable economic, social, physical, and psychological consequences of diagnosing diabetes. The current absence of a successfully implemented diabetes mass screening program is the result of the lack of resolution of these concerns (Harris et al. (1994) Diabetes Care. 17:440-445; Knowler (1994) Diabetes Care 17:445-450).

Current interest in diabetes screening programs have fostered studies on, for example, random capillary blood glucose measurements (Engelgau et al. (1995) Diabetes Care 18:463-466) and questionnaires to evaluate the prevalence of diabetes risk factors and thereby prospectively identify subjects at increased risk for undiagnosed diabetes (Herman et al. (1995) Diabetes Care 18:382-387). These tests have proven to be variable, empirical, and qualitative. Selecting those subjects from the general population who present risk factors merely identifies candidates for more precise and quantitative tests.

Accordingly, there is a need in the art for a more sensitive, more accurate, more acceptable, and standardized method for screening and/or diagnosis of diabetes and impaired glucose tolerance as well as a tool to assist in the management of disorders of carbohydrate metabolism.

Wolever et al. (Diabetes Care (1998) 21 336-340) and WO 97/02050 (Palmason and Wolever) describe the use of a solid oral diagnostic test meal for determining the postprandial concentration of a blood constituent in a vertebrate. The test meal bar disclosed in Wolever et al. comprises 41 g of starch, and 3.8 g. of total dietary fibre, while that disclosed in WO 97/02050 comprises 41-55 percent by weight of starch, and about 1.4 percent by weight of oat β-glucan. Both of these products have a high soluble fibre content.

It has been reported that the postprandial blood glucose response in subjects with normal or abnormal carbohydrate metabolism can be blunted by consumption of a high-carbohydrate, high-fibre bar (McIvor et al. (1985) Diabetes Care 8:274-278).
In further contrast, soluble dietary fibre has been reported to impair glucose absorption (see Wursch and Pi-Sunyer Diabetes Care: 20:1774-1780; Jenkins et al. European Journal of Clinical Nutrition 56 (7): 622-628 (2002)).

Certain forms of soluble dietary fibre significantly decrease post-prandial hyperglycaemia, by inhibiting glucose uptake from the small intestine into the blood, and may improve control of blood-glucose concentration by diabetics (Jenkins et al. Lancet 1976 Jul 24 2(7978):172-4). The diagnostic test meal of the present invention has a low soluble fibre content, containing less than 0.5 percent by weight.

The present invention also differs significantly from diabetic meals currently on the market which contain high fibre to improve control of blood glucose concentrations, such as the "CardioBar" (Abbott Laboratories). Other products contain mixed carbohydrate sources that release glucose over time, such as the "NiteBite"(ICN Pharmaceuticals, Inc.).

The product described in U.S. Patent No. 5,545,414 was developed by Abbott Laboratories and was trademarked as the *"CardioBar",* now marketed as the *"GlucernaBar"* as a snack for diabetics. The referenced patent describes a nutritional product developed specifically to lower cholesterol. The activity of the CardioBar product is attributable to dietary fibre, in this case guar gum, which affects cholesterol uptake in the gut. The preferred mode is to incorporate about 20 percent guar gum by weight into an unbaked food bar. The guar gum present in the CardioBar may impair glucose absorption from the small intestine into the blood, and result in readings of blood glucose concentrations that do not accurately reflect the degree to which cellular uptake of glucose is controlled. These readings can therefore result in diabetic patients being misdiagnosed as normal.

For the CardioBar, the preferred source of carbohydrate is high fructose corn syrup at approximately 24 percent by weight. In 1997, the FDA allowed health claims for the lowering of cholesterol by the inclusion of oats in a low fat diet. The CardioBar requirement of encapsulated dietary fibre (20 percent guar gum) is stated, as is the specification of low oil content.

The CardioBar human trials focused on the measurement of serum cholesterol, LDL, and HDL cholesterol. The method stated an overnight fasting and method of blood sampling, and methods for measuring serum cholesterol, LDL, and HDL concentrations. The patent utilized methods developed by Haber et al. for the measurement of satiety, plasma glucose, and serum insulin. Results relating to plasma glucose and serum insulin are not presented and indeed have not been published. The experimental results indicated that the guar gum present in the CardioBar had no effect on appetite or food intake.

U.S. Patent No. 4,496,606 by Michnowski describes a dietetic snack bar for use by type 2 diabetics in the regulation of glucose uptake for the control of impaired glucose tolerance and reduction of insulin requirement. The patent specifically cites the requirement of 8-12 percent guar gum, 27 percent corn syrup (glucose and fructose), and 6 percent simple sugar (selected from dextrose, fructose, glucose, and galactose). The product is designed specifically to pose a barrier through which nutrients must cross before being absorbed. When combined with a high simple carbohydrate (31 percent) a steady state blood carbohydrate level is obtained which is readily controlled by a diabetic; however, there may be a concern over administering such a product to a diabetic at risk of diabetic shock. The function of the invention described in U.S. Patent No. 4,496,606 by Michnowski, is supported by U.S. Patent No. 5,545,414, and that states that U.S. Patent No. 4,496,606 uses the consumption of guar gum to improve glucose tolerance and reduce insulin requirements (by virtue of the fact that glucose absorption through the small intestine into the blood is reduced).

Such a formulation is not applicable to a diagnostic product that requires the digestive function of the diabetic to breakdown starch into glucose, which is then absorbed in real-time for an accurate measurement of glycemic response.

U.S. Patent No. 5,612,074 by Leach describes a nutrient fortified, non-cooked, food bar consisting of 38 percent dietary fibres and approximately 30 percent honey or syrup (fructose, glucose). No measurement of glycemic response is included or considered. There may be a concern over administering such a product to a diabetic at risk of diabetic shock.

U.S. Patent No. 5,360,614 by Fox et al. describes the preparation of a composition for the slow release of carbohydrates by applying a coating of stearic acid, ethyl cellulose, or hydrogenated tallow. The result is the slow release of carbohydrate, peaking after 5 hours of digestion, which may be useful in sustained release.

U.S. Patent No. 6,248,375 by Gilles et al. describes solid matrix nutritionals designed for patients with diabetes. These nutritionals contain a source of fibre, and a combination of fructose with at least one nonabsorbent carbohydrate. The two component carbohydrate system is designed to blunt the postprandial glycemic response, and may be in the form of a bar.

Diabetologia vol. 20, pages 18-21 (1981) describes a study of the influence of low and high fibre diets upon carbohydrate tolerance in five maturity-onset, non-insulin dependent diabetics. After the high fibre test meal, significantly lower mean plasma glucose, insulin and gastric inhibitory polypeptide concentrations were measured. This study demonstrates the ability of an institutionally supervised diet of natural foodstuffs rich in fibre to improve carbohydrate tolerance in maturity-onset, non-insulin dependent diabetics.

Clinical Nutrition (1986) pages 209-212 describes a study examining the effect of the different fibre content of two commercial formulas on glycaemic and insulin response and hydrogen production in the colon during constant rate administration in 11 normal subjects. No difference in serum glucose and insulin levels was found. No rise in hydrogen production was detected with either formula suggesting no carbohydrate malabsorption. The quantity or nature of fibre present in blenderised formulas does not modify the pattern of carbohydrate absorption compared to a low-residue polymeric formula.

Am. J. Clin. Nutr. (2000) vol. 71, pages 1123-8 describes a study to determine whether arabinoxylan improves postprandial glucose and insulin responses in healthy humans. Arabinoxylan was the major component of dietary fiber in the cereal grains that make up a large proportion of the diet used in the study. Postprandial glucose and insulin response were found to be improved by ingestion of arabinoxylan-rich fiber.

It is an object of the present invention to overcome drawbacks of the prior art. The above object is met by a combination of the features of the main claims. The sub claims disclose further advantageous embodiments of the invention.

### SUMMARY OF THE INVENTION

The present invention relates generally to products for use in the detection of metabolic disease. In particular, the invention relates to a dry baked test meal useful in the screening and early diagnosis of impaired glucose tolerance and type-2 diabetes.

In a first aspect, the present invention provides a solid oral diagnostic test meal comprising:
(a) from 35 to 55 percent by weight of a glycemic polysaccharide;
(b) from 10 to 35 percent by weight mono- and disaccharides, the mono- and di-saccharides comprising glucose;
(c) from 10 to 25 percent by weight dietary fat, and
(d) from 5 to 8 percent by weight dietary protein,
   wherein the solid oral diagnostic test meal comprises less than 0.5 percent by weight soluble fibre and
   wherein the solid oral diagnostic test meal is in a dry form.

In another aspect, the present invention provides a use of the diagnostic test meal described herein for diagnosing a disorder of carbohydrate metabolism in a vertebrate subject.

In another aspect, the present invention provides a use of the oral diagnostic test meal described herein for determining a postprandial glucose concentration in a biological sample from a vertebrate subject.

In another aspect, the present invention provides a use of the oral diagnostic test meal described herein for determining a postprandial insulin response in a vertebrate subject.

In another aspect, the present invention provides a use of the oral diagnostic test meal described herein for diabetes self-diagnosis or self-monitoring in a vertebrate subject.

The present description describes an oral diagnostic test meal comprising a polysaccharide (complex carbohydrate), wherein the oral diagnostic test meal provides a quantity of glycemic carbohydrate effective to increase blood glucose levels in a vertebrate subject, and wherein the diagnostic test meal comprises less than 0.5 percent by weight soluble fibre.

The present description describes a method of diagnosing a disorder of carbohydrate metabolism in a vertebrate subject comprising:
(a) orally administering to the subject a diagnostic test meal comprising a polysaccharide, wherein the oral diagnostic test meal provides a quantity of glycemic carbohydrate effective to increase blood glucose levels in a vertebrate subject, and wherein the diagnostic test meal comprises less than 0.5 percent by weight soluble fibre;
(b) assaying a postprandial blood or plasma glucose concentration in the subject,
   and
(c) comparing the postprandial blood or plasma glucose concentration in the subject with a reference glucose concentration.

The disorder of carbohydrate metabolism may be selected from the group consisting of diabetes mellitus, impaired glucose tolerance, insulin resistance, non-insulin dependent diabetes, maturity onset diabetes, gestational diabetes and hyperinsulinemia.

The present description describes a method of determining a postprandial glucose concentration in a biological sample from a vertebrate subject comprising:
(a) orally administering to the subject a diagnostic test meal comprising a polysaccharide, wherein the oral diagnostic test meal provides a quantity of glycemic carbohydrate effective to increase blood glucose levels in a vertebrate subject, and wherein the diagnostic test meal comprises less than 0.5 percent by weight soluble fibre, and
(b) assaying a postprandial blood or plasma glucose concentration in the subject.

The present description describes a method of determining a postprandial insulin response in a vertebrate subject comprising:
(a) orally administering to the subject a diagnostic test meal comprising a polysaccharide, wherein the oral diagnostic test meal provides a quantity of glycemic carbohydrate effective to increase blood glucose levels in a vertebrate subject, and wherein the diagnostic test meal comprises less than 0.5 percent by weight soluble fibre, and
(b) assaying a postprandial blood or plasma insulin concentration in the subject

The present description describes a method of diabetes self-diagnosis and self-monitoring in a vertebrate subject, comprising:
(a) orally administering to the subject a diagnostic test meal comprising a polysaccharide, wherein the oral diagnostic test meal provides a quantity of glycemic carbohydrate effective to increase blood glucose levels in a vertebrate subject, and wherein the diagnostic test meal comprises less than 0.5 percent by weight soluble fibre, and
(b) assaying a postprandial blood or plasma glucose concentration in the subject.

The present description describes a method of managing the dosage of a drug that decreases postprandial blood glucose concentration in a vertebrate subject, comprising:
(a) orally administering to the subject a diagnostic test meal comprising a polysaccharide, wherein the oral diagnostic test meal provides a quantity of glycemic carbohydrate effective to increase blood glucose levels in a vertebrate subject, and wherein the diagnostic test meal comprises less than 0.5 percent by weight soluble fibre,
(b) assaying a postprandial blood or plasma glucose concentration in the subject,
   and
(c) repeating steps (a) to (b) after administration of the drug.

In the above-described methods, the postprandial glucose concentration can be determined from a biological sample, such as blood, taken from the subject.

The present description describes a method of calculating an average value of glycemic index of a test food, comprising:
(a) generating a first glucose response curve for the test food;
(b) generating a second glucose response curve for a reference food, wherein the reference food is a diagnostic test meal comprising a polysaccharide, wherein the oral diagnostic test meal provides a quantity of glycemic carbohydrate effective to increase blood glucose levels in a vertebrate subject, and wherein the reference food comprises less than 0.5 percent by weight soluble fibre,
(c) calculating a value of glycemic index of the test food from the first and second glucose response curves, and optionally
(d) repeating steps (a)-(c) for one, or more than one further subject, and determining the average value of glycemic index of the test food.

In an example of the above-described method of calculating, the first glucose response curve is generated by a process comprising:
(i) administering a portion of the test food to a subject, wherein the portion of the test food contains a standard amount of glycemic carbohydrate, and
(ii) measuring a blood or plasma glucose concentration in the subject at two, or more than two times following administration of the test food.

In a further example of the above-described methods of calculating, the second glucose response curve is generated by a process comprising:
(iii) administering a portion of the reference food to the subject, wherein the portion of the reference food contains a standard amount of glycemic carbohydrate, and
(iv) measuring a blood or plasma glucose concentration in the subject at two, or more than two times following administration of the reference food.

In a further example of the above-described methods of calculating, the step of calculating the glycemic index (step (c)) is conducted by dividing the area under the first glucose response curve by the area under the second glucose response curve.

In the above-described methods of calculating, the blood or plasma glucose concentration can be determined from a biological sample, such as blood, taken from the subject.

In another example of the above-described methods of calculating, steps (a)-(c) are repeated for at least nine further subjects.

The present description describes a method for the in-vivo diagnosis of a metabolic disorder, for example, diabetes and the pre-cursor of diabetes, impaired glucose tolerance, which comprises orally administering a calibrated amount of starch in a composition, allowing digestion to take place for between 30 to 60 minutes, and obtaining a blood sample for glucose estimation and analysis.

The present description describes a kit for diagnosing disorders of carbohydrate metabolism in a vertebrate subject, comprising an oral diagnostic test meal comprising a polysaccharide, wherein the oral diagnostic test meal provides a quantity of glycemic carbohydrate effective to increase blood glucose levels in a vertebrate subject, wherein the.diagnostic test meal comprises less than 0.5 percent by weight soluble fibre, and a suitable means for measuring blood glucose, such as a glucometer or a glucose-oxidase test strip.

The present description describes a kit for determining postprandial glucose concentration in a biological sample from a vertebrate subject, comprising an oral diagnostic test meal comprising a polysaccharide, wherein the oral diagnostic test meal provides a quantity of glycemic carbohydrate effective to increase blood glucose levels in a vertebrate subject, wherein the diagnostic test meal comprises less than 0.5 percent by weight soluble fibre, and a suitable means for measuring blood glucose, such as a glucometer or a glucose-oxidase test strip.

The present description describes a kit for determining a postprandial insulin response in a vertebrate subject, comprising an oral diagnostic test meal comprising a polysaccharide, wherein the oral diagnostic test meal provides a quantity of glycemic carbohydrate effective to increase blood glucose levels in a vertebrate subject, wherein the diagnostic test meal comprises less than 0.5 percent by weight soluble fibre, and a suitable means for measuring blood glucose, such as a glucometer or a glucose-oxidase test strip.

The present description describes a kit for diabetes self-diagnosis and self-monitoring in a vertebrate subject, comprising an oral diagnostic test meal comprising a polysaccharide, wherein the oral diagnostic test meal provides a quantity of glycemic carbohydrate effective to increase blood glucose levels in a vertebrate subject, wherein the Diagnostic test meal comprises less than 0.5 percent by weight soluble fibre, and a suitable means for measuring blood glucose, such as a glucometer or a glucose-oxidase test strip.

The present description describes a kit for managing the dosage of a drug that decreases postprandial glucose concentration in a vertebrate subject, comprising an oral diagnostic test meal comprising a polysaccharide, wherein the oral diagnostic test meal provides a quantity of glycemic carbohydrate effective to increase blood glucose levels in a vertebrate subject, wherein the diagnostic test meal comprises less than 0.5 percent by weight soluble fibre, and a suitable means for measuring blood glucose, such as a glucometer or a glucose-oxidase test strip.

In further examples, the oral diagnostic test meal described above contains less than 0.2 percent by weight soluble fibre.

The ratio of (a) to (b) in the just defined diagnostic test meal may be from about 1.5:1 to about 2.5:1, from about 1.8:1 to about 2.5:1, or from about 1.8:1 to about 2.0:1.

In an example of the above-defined diagnostic test meal, the polysaccharide is derived from a cereal grain selected from the group consisting of barley, oat, wheat, rye, corn, maize, sorghum and millet.

In another example, the above-defined diagnostic test meal further comprises one or both of a source of insoluble dietary fibre, and a source of flavoring.

In another example, the diagnostic test meal is provided in the form of a bar or biscuit.

In a further example, the polysaccharide of the diagnostic test meal described above may be derived from whole oat flour, defatted oat flour, or both.

In another example, the monosaccharide of the above-defined diagnostic test meal may be fructose, glucose, glycerin, or a mixture of glucose and fructose.

In a further example, the disaccharide of the above-described diagnostic test meal is sucrose.

The dietary fat of the diagnostic test meal described above may comprise from about 10 percent to about 30 percent saturated fat, and from about 25 percent to about 75 percent monounsaturated fat.

Diagnostic use of the test meal composition disclosed in WO 97/02050, or any other composition containing a significant amount of soluble fibre, can produce a viscous bolus within the small intestine of a patient, which contains glucose derived from digestion of the test meal composition. Due to the viscosity of the bolus, the amount and rate of glucose absorption into the blood of the patient is reduced. Diagnostic use of these compositions may, therefore, result in a measured value of glycemic response in the patient, which does not accurately reflect the degree to which the cellular uptake of glucose is being successfully regulated. For example, measurement of the glycemic response in a diabetic following administration of a composition comprising a significant amount of a soluble fibre, may provide a blood glucose concentration that is typical of a normal person. The use of these compositions in a diagnostic method can, therefore, lead to misdiagnosis of a diabetic subject or a subject having impaired glucose tolerance as being normal.

The test meal composition of the present invention comprises less than 30 percent of the total amount of soluble fibre contained in the diagnostic test meal disclosed in WO 97/02050, i.e., less than 0.5 percent by weight soluble fibre. During the process of digestion of the composition of the present invention, the relatively lower amount of soluble fibre present in the composition will not significantly change the viscosity of fluid within the digestive tract, and will not, therefore, significantly affect the amount and rate of glucose absorption through the wall of the small intestine. As a result, the composition of the present invention can be used to determine a glycemic response of a patient, which accurately reflects the degree to which the cellular uptake of glucose is being successfully regulated.

The present invention has a shelf life of approximately two years, is consistent in ingredients and can be reproducibly manufactured. Furthermore, standard chemical and laboratory methods can be used in analyzing the constituents of the diagnostic test meal of the present invention for the purposes of quality assurance and quality control. Quality assurance and quality control testing of the diagnostic test meal of Wolever et al. (Diabetes Care (1998) 21 336-340), however, is dependent on *in-vivo* glycemic indexing.

As the test meal composition of the present invention comprises a complex carbohydrate, which is gradually digested into glucose, the risk of diabetic shock associated with its consumption is low. The selected source of starch is of such purity that a calibration in terms of glucose equivalents is possible.

This summary does not necessarily describe all necessary features of the invention but that the invention may also reside in a sub-combination of the described features.

### BRIEF DESCRIPTION OF THE DRAWINGS

These and other features of the invention will become more apparent from the following description in which reference is made to the appended drawings wherein:

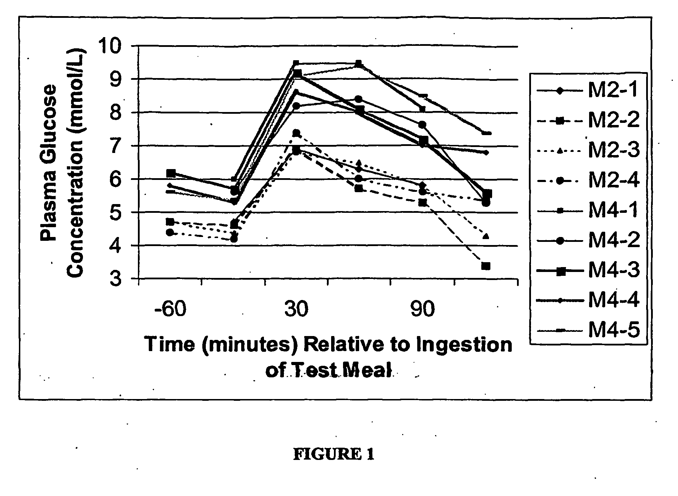

Figure 1 illustrates the glycemic response to the diagnostic test of the present invention for a normal patient, and a patient suffering from impaired glucose tolerance (IGT). The points represent five or four separate tests conducted on the normal or IGT patient, respectively.

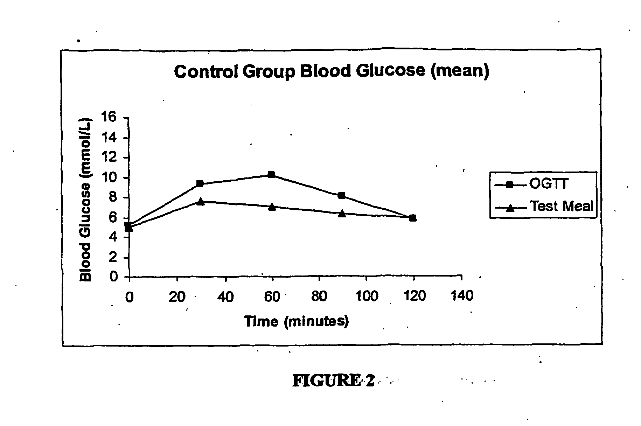

Figure 2 illustrates the mean glycemic responses of eleven non-obese, normal patients ("Control Group") determined using both the oral glucose tolerance test and the diagnostic test meal of the present invention. Each data point for the different tests reflects the mean value of blood glucose concentration of the patients at a particular time.

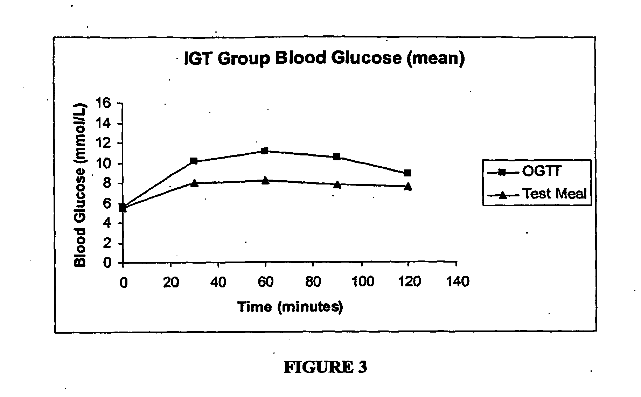

Figure 3 illustrates the mean glycemic responses of five impaired glucose tolerant patients ("IGT group") using both the oral glucose tolerance test and the diagnostic test meal of the present invention. Each data point for the different tests reflects the mean value of blood glucose concentration of the patients at a particular time.

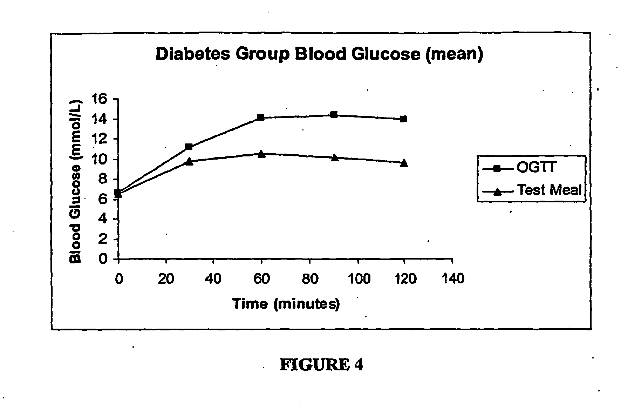

Figure 4 illustrates the mean glycemic responses of thirteen non-insulin dependent (type 2) diabetic ("Diabetes Group") patients using both the oral glucose tolerance test and the diagnostic test meal of the present invention. Each data point for the different tests reflects the mean value of blood glucose concentration of the patients at a particular time.

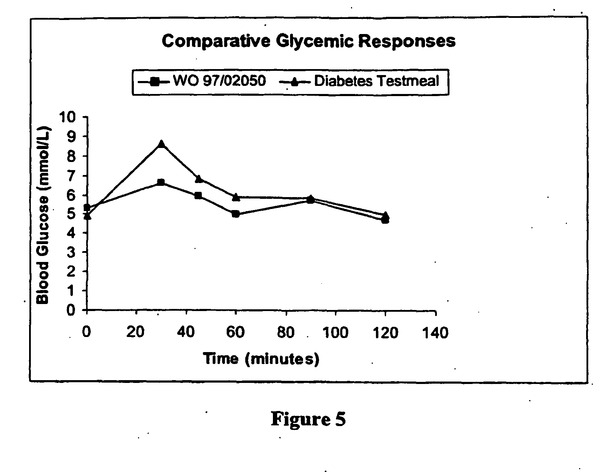

Figure 5 illustrates the comparative glycemic responses of the diagnostic diabetes test meal of the present invention and the test meal described in WO 97/02050. The glycemic responses plotted are for the same nonobese, normal subject.

### DETAILED DESCRIPTION OF THE INVENTION

The present invention relates generally to products for use in the detection of metabolic disease. In particular, the invention relates to a dry baked test meal useful in the screening and early diagnosis of impaired glucose tolerance and type-2 diabetes.

Central to the present invention is the discovery that the novel dry screening and/or diagnostic test meal disclosed herein produces a more precise assessment of an individuals' health with respect to diabetes or impaired glucose tolerance.

Accordingly, the present invention provides an oral diagnostic test meal for use in the screening and early diagnosis and management of diabetes.

The practice of the present invention will employ, unless otherwise indicated, conventional methods of chemistry, biochemistry, medicine, nutritional analysis, and food formulation, within the skill of the art. Such techniques are explained fully in the literature. *See,* e.g., Bergmeyer et al., eds. Methods of Enzymatic Analysis, Academic Press (New York), U.S. Dept. HEW (1982) Lipid and Lipoprotein Analysis: Manual of Laboratory Operations, Lipid Research Clinics Program, (Washington, DC), AOAC (1980) Official Methods of Analysis (Washington, DC), National Diabetes Group (1979) Classification and Diagnosis of Diabetes Mellitus and Other Categories of Glucose Intolerance, Diabetes 28:1039-1057, Furia (1972 and 1980) Handbook of Food Additives, 2d ed., Volumes I and II, CRC Press Inc. (West Palm Beach, FL), and Committee on Specifications, Committee on Food Protection, National Research Council (1980) Food Chemicals Codex, 3d ed., National Academy of Sciences (Washington, DC).

As used in this specification and the appended claims, the singular forms "a", "an", and "the" include plural references unless the content clearly dictates otherwise. Thus, the term "a blood glucose measurement" can include more than one such measurement.

In describing the present invention, the following terms will now be employed, and are intended to be defined as indicated below. Although reference is made to blood as a biological sample in the following definitions, the term is intended to encompass other biological samples as well.

By "dry form" is meant a product which has been baked to a dry form and has a moisture content of <5 percent.

By "glycemic response" is meant the amount of glucose produced in the blood following ingestion of a food, test meal, or the like taken at a specific postprandial time point, for example, 30, 60, 90, and/or 120 minutes.

The "glycemic index" may be used as a basis for dietary carbohydrate exchange and as a reference for assessing the glucose response to a particular food. (*See,* Jenkins et al. (1981) Am. J. Clin. Nutr. 34:362-366, and Jenkins et al. (1983) Diabetologia 24:257-264). The glycemic index is defined as the glycemic response elicited by a test food containing 50 g of glycemic carbohydrate, expressed as a percentage of the glycemic response elicited by a reference food containing 50 g of glycemic carbohydrate. The reference food can either be 50 g of glucose, usually in the form of a concentrated solution, or, alternatively, a portion of freshly baked white bread containing 50 g of glycemic carbohydrate. A portion of white bread containing 50 g of glycemic carbohydrate raises blood glucose levels 71 percent as much as 50 g of glucose. Nevertheless, if either 50 g of glucose or a portion of white bread containing 50 g of glycemic carbohydrate is used as the reference food, each is defined as having a glycemic index of 100.

The glycemic index may be determined as described in Wolever et al. (1985) Diabetes Care 8:418-428, Wolever et al., Journal of the American College of Nutrition 8(3):235-247 (1989), Wolever et al. (1991) Am. J. Clin. Nutr. 54:846-854, and Wolever et al. (1994) Am. J. Clin. Nutr. 59:1265-1269. Generally, the glycemic index of the test food can be measured *in vitro* or *in vivo.* Although the *in vitro* methodology is rapid and inexpensive, it cannot totally replicate the complex processes of human digestion. The *in vivo* methodology involves administering the test food in at least ten healthy human subjects following an overnight fast. A 50g glycemic carbohydrate portion of the test food is consumed and blood glucose levels are measured over the next two hours. On a different occasion, after an overnight fast, 50g of glucose, in the form, for example, of a concentrated glucose solution, or a portion of white bread containing 50 g of glycemic carbohydrate is consumed, and the blood glucose levels measured over the next two hours. The glycemic index is determined by dividing the incremental area under the glucose response curve of the test food by the incremental area under the glucose response curve of the reference food. The final glycemic index value is the average glycemic index of the ten healthy subjects.

By "available carbohydrate" is meant the amount of total carbohydrate ingested, which can be digested and absorbed. Total carbohydrate is calculated by difference, i.e., total carbohydrate ("TC") is calculated using the equation TC = S - M - A - F - P, where S is the weight of the food sample, M is the moisture content, A is the ash content, F is the fat content, and P is the protein content. The methods for measuring moisture, ash, fat, and protein are described in, e.g., AOAC Official Methods of Analysis (1980) Washington, DC, Association of Official Analytical Chemists. Available carbohydrate is the difference between total carbohydrate and dietary fibre in a food sample. Dietary fibre can be measured, e.g., according to the method of Prosky et al. (1988) J. Assoc. Off. Anal. Chem. 71:1017.

By "glycemic carbohydrate" is meant glucose or a complex carbohydrate, such as a glycemic polysaccharide, e.g. starch, which can be metabolized or reduced to glucose. Digestion and absorption of glycemic carbohydrate has a measurable effect on an individual's blood glucose concentration.

By "management of disorders of carbohydrate metabolism" it is intended that the test may be used to establish and manage drug dosage.

By "medically controlled" is meant that a diagnostic test meal contains a source of carbohydrate that provides a standardized and calibrated amount of glycemic carbohydrate which, when administered to a subject, yields a glycemic response in that subject. The present test does not require periodical recalibration as it delivers a standardized quantity of carbohydrate in each test meal.

A "reference concentration" of glucose in a biological specimen is that concentration that has been reported as normal for a non-obese, non-diabetic, healthy subject. The reference concentration may be in reference to fasting glucose or in reference to postprandial glucose concentration. Such reference concentrations are described in, for example, National Diabetes Group (1979) Diabetes 28:1039-1057, and WHO Expert Committee (1980) Diabetes Mellitus, Geneva, World Health Organization (Tech. Rep. Ser. No. 646). In addition, a reference concentration may be an intra-subject reference, i.e., a concentration of glucose or insulin in a biological sample previously obtained from the subject being retested.

By "vertebrate subject" is meant any member of the subphylum chordate, including, without limitation, mammals such as cattle, sheep, pigs, goats, and man; domestic animals such as dogs and cats; and birds, including domestic, wild and game birds such as cocks and hens including chickens, turkeys, and other gallinaceous birds. The term does not denote a particular age. Thus, both adult and newborn animals are intended to be covered.

The terms "self-diagnosis", "self-teaching", and "self-monitoring" are meant to encompass the use of the diagnostic test meal in a non-clinic setting. Thus, for example, self-diagnosis is intended to encompass not only the use of the diagnostic test meal by a human subject on himself or herself for the diagnosis of a disorder of carbohydrate metabolism, but also the use of the diagnostic test meal by a party for the diagnosis of a disorder of carbohydrate metabolism in another vertebrate subject.

A "biological sample" may be derived from a variety of sources, e.g., human or other mammalian biological fluids or tissues, including blood (serum or plasma), urine, cerebrospinal fluid, lymph fluid, and the like.

The terms "blood glucose" is intended to mean glucose, measured in whole blood, serum, or plasma taken from a vein or a capillary bed. Typical sources of capillary blood include finger, heel, or earlobe-pricks. One of ordinary skill in the art will recognize that the blood glucose level will vary slightly depending on the source.

The glucose levels in a biological sample may be measured by any method known in the art. In this regard, glucose levels may be measured using the hexokinase method (Bergmeyer et al. (1974) in Bergmeyer et al., eds. Methods of Enzymatic Analysis, Academic Press (New York)). Briefly, this assay entails simultaneously incubating the biological sample with the enzyme hexokinase, which catalyzes the transfer of the γ-phosphate group from adenosine triphosphate ("ATP") to glucose to form glucose-6-phosphate and glucose-6-phosphate dehydrogenase, which, in the presence of nicotinamide-adenine dinucleotide phosphate ("NADP"), catalyzes the conversion glucose-6-phosphate to 6-phosphogluconate and reduced NADP ("NADPH"). The resultant NADPH is coupled to the reduction of iodonitrotetrazolium ("INT"), or other reagent which when chemically reduced forms a colorimetrically detectable species, to form INT-formazan.

Optionally, glucose may be measured, e.g., using glucose oxidase-catalyzed conversion of glucose to gluconic acid, thereby producing hydrogen peroxide, which may be detected colorimetrically by, for example, incubation with 4-aminoantipyrine and *p-*hydroxybenzene sulfonate or *o*-dianisidine in the presence of peroxidase to produce the colorimetrically detectable species quinoneimine dye or oxidized o-dianisidine, respectively. By comparison with a standard curve generated using known quantities of glucose, the amount of glucose in the sample can be determined.

The individual components required for the above-described glucose assays, as well as kits therefore, may be obtained from any commercial source, e.g., Sigma Chemical Co. (St. Louis, MO).

The dry carbohydrate diagnostic test meal disclosed and claimed herein contains a carbohydrate source providing an amount of a polysaccharide (complex carbohydrate), such as starch, which has been calibrated and standardized to provide a selected quantity of glycemic carbohydrate upon ingestion of the test meal by a vertebrate subject. A particular example of a polysaccharide is oat starch formulated with a minimum quantity of β-glucan from oat grain, or another cereal grain including, without limitation, any of the various cultivars of e.g., barley, oat, wheat, rye, corn, maize, sorghum, and millet, or another source, such as potato, sweet potato, canna, arrowroot, tapioca (cassava) sago, arum, triticale, rice, amaranth, quinoa, beans, peas, lentils, chestnuts, peanuts, inulin, lichen, or the like. Alternatively, any synthetic starch well known in the art can be included as a carbohydrate source. Other ingredients may also be included such as protein, fat, texture, and palatability enhancers and the like.

The present invention contains a physiologically balanced carbohydrate source, but need not contain the recommended dietary requirements for carbohydrate, fat and protein. The recommended daily requirements for diabetics is the same as those for normal people, with 30 percent of the daily energy requirements from fats, 10-20 percent from proteins and 40-50 percent from carbohydrates (American Diabetes Association).

In particular, the composition of the present invention has a soluble fibre content of less than 0.5 percent by weight, or more particularly less than 0.2 percent by weight. As a result, administration of the composition of the present invention to a subject will not significantly increase the viscosity of fluid within the small intestine, and, therefore, not result in a significant reduction in glucose absorption through the small intestine and into the blood of the subject

The diagnostic test meal of the present invention may contain from about 45 to about 55 percent by weight, from about 45 to about 50 percent by weight, or any value or subrange between these ranges, of a polysaccharide (complex carbohydrate).

The test meal may also contain from about 10 to about 35 percent by weight, from about 10 to 30 by weight, from about 10 to 25 percent by weight, from about 10 to 20 percent by weight, or any value or subrange between these ranges, of mono- and disaccharides.

In addition, the test meal may contain from about 10 to about 20 percent by weight, from about 15 to about 25 percent by weight, from about 15 to about 20 percent by weight, or any value or subrange between these ranges, of fat, of which from about 10 percent to about 30 percent is saturated fat, from about 25 percent to about 75 percent or any value or subrange therebetween, or from about 45 percent to about 55 percent or any value or subrange therebetween, is monounsaturated fat, and the remainder is polyunsaturated fat.

The test meal of the present application also contains from about 5 to about 8 percent by weight or any value or subrange therebetween, of protein, and optionally about 3 to 5 grams, or any value or subrange therebetween, of total dietary fibre.

In addition, the diagnostic test meal of the present invention may contain from about 45 to 55 percent by weight, or any value or subrange therebetween, of glycemic carbohydrates.

The ratio of the amount of the polysaccharide (expressed in percent by weight) to the sum of the amounts of the monosaccharide and the disaccharide (each expressed in percent by weight) may be from about 1:1 to about 2.8:1 or any value or subrange therebetween, from about 1.3 to about 2.8:1 or any value or subrange therebetween, from about 1.4 to about 2.5:1 or any value or subrange therebetween, from about 1.5:1 to about 2.5:1 or any value or subrange therebetween, from about 1.7:1 to about 2.5:1 or any value or subrange therebetween, from about 1.8:1 to about 2.5:1 or any value or subrange therebetween, from about 1.8:1 to about 2.3:1 or any value or subrange therebetween, or from about 1.8:1 to about 2.0:1 or any value or subrange therebetween. In other examples, the ratio of the amount of polysaccharide to the sum of the amounts of monosaccharide and disaccharide is from about 1.5:1 to about 2.0:1 or any value or subrange therebetween, or from about 1.5:1 to about 1.8:1 or any value or subrange therebetween.

The ratio of the amount of the polysaccharide to the amount of protein (each expressed in percent by weight) may be from about 1.5:1 to about 9.0:1 or any value or subrange therebetween, from about 1.7:1 to about 4.5:1 or any value or subrange therebetween, from about 1.8:1 to about 4.0:1 or any value or subrange therebetween, from about 1.8:1 to about 3.5:1 or any value or subrange therebetween, or from about 1.8:1 to about 3.0:1 or any value or subrange therebetween.

The ratio of the amount of the polysaccharide to the total amount of fat (each expressed in percent by weight) may be from about 1.5:1 to about 6.0:1 or any value or subrange therebetween, from about 1.7:1 to about 4.5:1 or any value or subrange therebetween, from about 1.8:1 to about 4.0:1 or any value or subrange therebetween, from about 1.8:1 to about 3.5:1 or any value or subrange therebetween, or from about 1.8:1 to about 3.0:1 or any value or subrange therebetween.

Furthermore, the test meal of the present application may have a ratio of total glycemic carbohydrates to total carbohydrates (each expressed in percent by weight) from about 1:2 to about 1:1.25 or any value or subrange therebetween, or from about 1:1.67 to about 1:1.25 or any value or subrange therebetween.

Examples of fat used in the test meal compositions of the present invention is fat obtained from vegetable oils, such as canola oil, rapeseed oil, flax oil, borage oil, safflower oil, soybean oil, coconut oil, evening-primrose oil, castor oil, olive oil, almond oil, peanut oil, linseed oil, corn oil, and maize oil, fish oils such as cod liver oil or halibut oil, extracted oat fat or the like, protein obtained from soy flour, egg albumin, ovoglobulin, wheat gluten, whey, lactoglobulin, lactalbumin, meat and blood isolates, serum albumin, fish protein isolates, legume isolates, soy protein, quinoa protein, or the like.

In order to increase the palatability of the test meal, flavoring such as apple juice, cinnamon, vanilla, lemon, or orange extracts, almond flavor, or the like may be added to the test meal. Other standard food additives may be incorporated into the test meal, for example, acidulants, anticaking agents, baking aids, bleaching agents, buffering agents, colorants, color fixatives, cooking media, dough conditioners, emulsifiers, enzymes, firming agents, flavor enhancers, flavors, humectants, leavening agents, masticatory substances, maturing agents, neutralizers, nonnutritive sweeteners, nutrients and dietary supplements, preservatives, protein sources, salt substitutes, sequestrants, stabilizers, sweeteners, texturizers, thickeners, vitamins, and the like. *See,* e.g., Furia (1972 and 1980) Handbook of Food Additives, 2d ed., Volumes I and II, CRC Press Inc. (West Palm Beach, FL).

An example of a test meal, which provides 406.5 kcal of energy, comprises 50.0 grams of carbohydrate (49 percent of the meal energy) comprising starch extracted from oat groats, 19.9 grams of fat (44 percent of the meal energy) comprising vegetable and oat fat, 6.69 grams of protein (7 percent of the meal energy) comprising protein derived from oat flour and egg albumin, 4.30 grams of total dietary fibre comprising β-glucan derived from whole oat flour, and vanilla and butter flavors for flavoring.

The amount of glycemic carbohydrate in the test meal may be calibrated to achieve a selected glycemic response. A reference glycemic response may be obtained using, for example, an oral liquid glucose standard containing a quantity of glucose, for example 50 grams, 75 grams, or 100 grams of glucose. The amount of glycemic carbohydrate in a test meal may be standardized to achieve a selected 30-minute, 45-minute, 60-minute, 90-minute, 120-minute, or the like, postprandial blood glucose response. For a test meal calibrated against an oral liquid glucose reference, the reference glycemic response may be determined using a 75-gram oral liquid glucose beverage, e.g., Glucodex^{®}.

The polysaccharide may be prepared from cereal grains, such as oats, using the method disclosed in U.S. Patent No. 4,435,429, issued to Burrows et al, . Briefly, the process involves separation of endospermic tissue from other tissues in grain by first soaking the grain in an aqueous medium, pH 3-7, at 400°C to 700°C until the grain has absorbed at least its own weight of the aqueous medium and the endosperm portion of the grain has been liquified by the action of enzymes indigenous to the cell wall. The grain is then split under pressure to release virtually all the liquified endosperm. The endosperm can then be separated from the other tissues of the grain. The grain can be whole, dehulled or hulless. The aqueous medium may contain up to about 0.1 wt. percent SO₂. Using this method, low fibre, off-white flour can be produced in the wet or dry state ready for further fractionating.

Optionally, the polysaccharide is derived from cereal grains, such as oat grains, which may be prepared using the method described in U.S. Patent No. 5,169,660, issued to Collins et al. The method involves first steeping the cereal grain in water for a period of time sufficient to substantially completely liquify the endosperm. The steeped grain is then macerated in an aqueous ethanol solution to liberate the liquid endosperm.

Insoluble bran is then separated and recovered from the aqueous ethanol solution and the insoluble flour is separated and recovered from the bran-free aqueous ethanol solution. Recovery of the insoluble bran and flour from the aqueous ethanol solution may be effected by passing the solution over sequentially finer mesh screens. This method is particularly useful for producing relatively pure bran and flour from oat, wheat and rye grains.

The test meal may be in any solid physical form such as a bar, wafer, biscuit, cookie, or the like. In one example, the test meal is in the form of cookies, each of which can deliver 5 grams glycemic carbohydrates. The meal can be standardized for 50 grams or 75 grams glycemic carbohydrates, or administered on a per kilogram of patient's body mass.

The complex carbohydrate within the composition of the present invention must be digested before glucose is released and absorbed. Since digestion will be specific to the test subject, the release and absorption of glucose will be specific for that individual. The absorption process will also take place in the context of a normal, solid meal and the kinetics of glucose absorption will relate to "normal" physiological events rather than a test or specially modified meal.

As a result, the blood glucose determinations with the test meal composition of the present invention will be accurate and relate to an individual's normal physiology. This allows for the rapid detection of the diabetic state, often at a time before full-blown diabetes is established, allowing for early remedial actions to be taken.

The test meal of the present invention may be made by routine methods well known to those of skill in the art. For example, the components of the test meal may be blended in predetermined proportions with a binding agent, if necessary, selected so as to preserve the component proportions of the product, e.g., whole egg powder, or wheat gluten, to form a mixture. The mixture may be molded, pressed, poured, extruded, or otherwise formed into a desired shape. The shaped product may also be baked, steamed, dried, or otherwise processed, as required, to set the shape. *See,* e.g., U.S. Patent No. 5,200,215 to Slade et al.

The test meal disclosed herein produces a more precise (CV ≈ 3 percent-5 percent) and more physiological measurement of postprandial glucose response compared to liquid glucose beverages such as Glucodex^{®}. Furthermore, the dry test meal produces more precise responses than liquid meals such as Enrich^{®} (Ross Laboratories, Montreal, Canada). Thus, in using the dry test meal, it is possible to predict blood glucose response with only 3 percent-5 percent error. Accordingly, the dry test meal can be used as a substitute for the liquid 75-gram liquid glucose standard for the diagnosis and/or management of diabetes and impaired glucose tolerance or for the regulation of hypoglycemia. In addition, the test meal can be used to determine postprandial glucose response.

Conventional fasting blood glucose measurements are unreliable for the assessment of disorders of carbohydrate metabolism because as many as 39 percent of the subjects in the upper range of normal fasted glucose levels show diabetic postprandial glucose responses. Thus, they can only be found with a 60-minute or greater postprandial carbohydrate challenge. Using the novel dry test meal disclosed herein, a postprandial glucose response assessment can be initiated 30-45 minutes before clinical laboratory examination, e.g., a subject ingests the test meal 30-45 minutes before blood is drawn at the clinic. In addition, the test meal can be used for self-diagnosis, self-teaching, and blood glucose self-monitoring.

In addition, the diagnostic test meal can be used in the management of disorders of carbohydrate metabolism. In particular, the test meal can be used as a standard test meal to titrate drug dosage for the treatment of these diseases in individual subjects with oral antidiabetic agents. For example, α-glucosidase inhibitors, e.g., acarbose, are a class of drugs which improve blood glucose control in diabetics (Clissold et al. (1988) Drugs 35:214-243; Chiasson et al. (1994) Ann. Int. Med. 121:928-935). Following a liquid test meal, acarbose treatment causes a decrease in the mean glucose peak determined 90 minutes postprandial in patients with NIDDM (Chiasson et al., supra). However, flatulence, abdominal distension and diarrhea are major side effects associated with α-glucosidase inhibitors. Thus, it is desirable to titrate the dose of acarbose to that dose required to suppress postprandial plasma glucose.

The diagnostic test meal disclosed herein also affords a method by which such dosing regimens can be titrated to an optimal level. In order to assess the dosage of drugs required to decrease postprandial blood glucose a predetermined amount, a solid test meal may be administered to a subject and plasma glucose levels monitored at 0 (i.e., the 10 to 16-hour fasting level) 30, 45, 60, 90 and 120 minutes. The incremental area under the glycemic response curve is calculated geometrically, ignoring the area beneath the fasting value (Wolever et al. (1991) Am. J. Clin. Nutr. 54:846-854). This procedure is repeated after administration of the antidiabetic agent to determine if the drug produces the desired diminution in glycemic response. The dose of drug administered is then adjusted upward or downward as necessary to achieve the desired effect. The process may be repeated as often as required until the desired drug response is achieved. In addition, the efficacy of the treatment may be assessed periodically and the drug dosage adjusted as necessary.

A determination of the efficacy of an antidiabetic drug, and whether adjustment of the drug dosage is required, may be made by monitoring changes in the postprandial blood glucose concentration following administration of the drug. Acceptable limits of a drug-induced change in postprandial blood glucose concentration as indicating either acceptable efficacy or a necessity for further titration of the dosage are well known to those of skill in the art.

For example, it is generally accepted that, in a subject that has been treated with a first dose of an antidiabetic agent, a second higher or lower dose of the agent that respectively effects a decrease or increase in blood glucose of approximately 2 mmol/L is considered equally efficacious.

In individuals with diabetes mellitus, control of blood glucose levels is impaired. Either insulin production is decreased or absent, or the ability of the body to utilize insulin is defective. In the absence of insulin, or the impaired ability of insulin to move glucose into cells, consumption of glycemic carbohydrates, and subsequent absorption of glucose, entails that glucose remains in the blood for longer periods of time than is normal. These elevated blood glucose levels are the cause of many of the complications associated with diabetes, such as heart disease, strokes, nerve and kidney damage etc.

Diabetics are highly sensitive to the amount of carbohydrate ingested due to the resultant elevated blood glucose levels. Precise control of blood glucose levels is required. To make this task easier, the glycemic index of foods are determined so that diabetics can estimate what their resultant blood glucose levels will be after eating certain foods.

As indicated above, white bread may be used as the reference food for calculating the glycemic index of a test food. Although the bread is baked from weighed ingredients, the composition of these ingredients may vary between batches. The composition of the bread will also vary depending on where it is baked. Therefore, there is little global uniformity or standardization available with white bread.

As an alternative to using white bread, the standardized test meal of the present application could be used as the reference food. The ingredients of the test meal are highly standardized and quality controlled, and each batch is essentially identical. Different glycemic index testing laboratories could use the test meal to ensure continuity and, therefore, as a global standard reference meal. Since the test meal can be produced in portions containing 5 g of glycemic carbohydrate, there is no necessity to utilize a 50 g standardized meal. Portions of the test meal can also be selected to deliver a smaller dose. This may be more in keeping with the average serving sizes of different foods and easier for comparative purposes.

The test meal of the present application can, therefore, be used to develop indexed foods, and may also be used as a standardized reference in the development of products containing grains and sugars, not only for humans, but also for pets and livestock.

An additional use for the dry test meal is in the area of self-diagnosis and self-monitoring. Using currently available techniques well known to those in the art, a subject can obtain fasting and postprandial blood samples which can be analyzed for glucose levels using, for example, the Bayer Glucometer Elite Diabetes Care System (Bayer AG, Leverkusen, Germany), LifeScan SureStep Blood Glucose Monitoring System (Lifescan, Inc., Milpitas, California), and Roche Accu-Chek Instant Blood Glucose Meter (Roche Diagnostics, Basel, Switzerland).

Referring to Figure 1, there is shown the glycemic response to the test meal of the present invention for a normal, and a patient suffering from impaired glucose tolerance (IGT). It is clear that a glycemic response can be measured within 30-45 minutes of consuming the test meal. The response varies according to the status of the patient taking the test, and is indicative of their diagnosis. The points are the average of four or five separate tests conducted on the IGT and the normal patient, respectively.

It is to be understood that while the invention has been described in conjunction with the preferred specific embodiments thereof, that the description above as well as the examples, which follow are intended to illustrate and not limit the scope of the invention.

The following examples are put forth so as to provide those of ordinary skill in the art with a complete disclosure and description of how to make and use the compounds of the invention, and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C, and pressure is at or near atmospheric.

### Materials and Methods:

Finger-prick capillary blood samples (≈ 200 µl) were collected into fluoro-oxalate tubes and stored at -20°C for a maximum of 24 hours. Whole blood glucose was measured using a model 2300 STAT glucose analyzer (Yellow Springs Instruments, Ohio). Incremental areas under the glycemic response curves ("AUC") were calculated geometrically, ignoring area beneath the fasting value (Wolever et al. (1991) Am. J. Clin. Nutr. 54:846-854).

Plasma glucose was measured using the hexokinase method (Bergmeyer et al. (1974) in Bergmeyer et al., eds. Methods of Enzymatic Analysis, Academic Press (New York)).

All procedures involving human subjects were approved by the University of Alberta Ethics Committee or other appropriate review committee.

### EXAMPLE 1. Solid Oral Carbohydrate Diagnostic Test Meal

The following components were combined in the amounts indicated below in Tables 1 and 2 to produce a wet product for use in preparing the test meal composition

**Table 1. Batter composition used for preparing test meal from WO 97/02050 (Comparative)**

| Ingredient | Weight (grams) | Weight % total |
|---|---|---|
| Water | 44 | 33.02 |
| Rolled oat flakes | 19.82 | 14.88 [9.82 polysaccharide (glycemic carbohydrate), (A)] |
| Pin Milled Flour | 13.21 | 9.91 [6:54 polysaccharide (glycemic carbohydrate), (B)] |
| Pin Milled Starch | 13.21 | 9.91 [8.52 polysaccharide (glycemic carbohydrate), (C)] |
| Oat Bran | 6.60 | 4.95 |
| Liquid Honey | 9.55 | 7.17 [5.59 mono and di-saccharides (D); 2.15 glucose (glycemic carbohydrate), (E)] |
| Canola Oil | 7.78 | 5.84 |
| Soy Protein | 5.73 | 4.3 |
| Sugar | 3.69 | 2.77 (F) [1.38 glucose (glycemic carbohydrate), (G)] |
| Glycerin | 3.34 | 2.51 |
| Gluten | 2.86 | 2.15 |
| Baking Powder | 2.19 | 1.65 |
| Cinnamon | 1.10 | 0.82 |
| Salt | 0.12 | 0.09 |
| All spice | 0.04 | 0.03 |
| Total weight | 133.24 | |
| Total wt. % glycemic | | |
| carbohydrate | | |
| (A+B+C+E+G), I | 28.41 | |
| Total wt. % carbohydrates | | |
| (A + B + C + D + F), II | 33.24 | |
| I/II | 0.85 | |
| Total wt.% polysaccharides (glycemic carbohydrate) (A + B + C), III | 24.88 | |
| Total wt. % mono- and di-sacharides (D + F), IV | 8.36 | |
| III/IV | 2.97 | |
| Total wt. % glycemic carbohydrate in final product produced from batter | 39.76 | |
| Weight % soluble fiber in final product produced from batter | A minumum of 1.5 wt. % | |

**Table 2. Batter composition used to form test meal bar of the present invention**

| Ingredient | Weight (grams) | Weight. % total |
|---|---|---|
| Sugar-granulated | 110 | 8.1 (C) [4.05 glucose (glycemic carbohydrate), (F)] |
| Fructose | 135 | 9.94 (D) |
| Baking Soda | 6.6 | 0.49 |
| Baking Powder | 8.8 | 0.65 |
| Butter Flavour | 3.1 | 0.23 |
| Vanilla Flavour | 1 | 0.07 |
| Salt | 1.76 | 0.13' |
| Whole Oat Flour | 441 | 32.47 [21.43 polysaccharide (glycemic carbohydrate), (A)] |
| Defatted Oat Flour | 244 | 17.97 [14.38 polysaccharide (glycemic carbohydrate), (B)] |
| Whey Powder | 29.9 | 2.2 |
| Shortening | 129.3 | 9.52 |
| Invert Sugar | 129.3 | 9.52 [7.33 glucose-fructose (E); 3.66 glucose (glycemic carbohydrate), (G)] |
| Soy Lecithin | 5.71 | 0.42 |
| Liquid Whole Egg | 56.3 | 4.15 |
| Water | 56.3 | 4.15 |
| Total Weight | 1358.07 | |
| Total wt. % glycemic carbohydrate (A+B+F+G),I I | 43.52 | |
| Total wt. % carbohydrates (A+B+C+D+E), II | 61.18 | |
| I/II | 0.71 | |
| Total wt. % polysaccharides (glycemic carbohydrate) (A + B), III . | 35.81 | |
| Total wt. % mono- and di-sacharides (C+D+E),IV | 25.36 | |
| III/IV | 1.41 | |
| Total wt. % glycemic carbohydrate in final product produced from batter | 50.5 | |
| Weight % soluble fiber in final product produced from batter | Less than 0.5 wt. % | |

A typical analysis of a 100-gram solid oral diagnostic test meal of the preset invention is given in Table 3 below.

**Table 3:**

| | Best Mode for 50 grams glycemic carbohydrate | Source |
|---|---|---|
| Meal Weight | 100 grams | |
| Glycemic Carbohydrate | 50.0 grams (200 kcal; 49% of total energy) | oat starch, oat flour, and sugars |
| Fat | 19.9 grams (179 kcal; 44% of total energy) | whole oat flour and shortening |
| Protein | 6.69 grams (27 kcal; 7% of total energy) | oat flour and egg albumin |
| Soluble Fibre | <0.5 grams soluble fibre, such as β-glucan) | oat flour |
| Flavoring | | vanilla and butter flavors |

### Example 2. Glycemic Responses to the Test Meal

One normal (M2) and one IGT subject (M4), each in the fasted state, consumed the test meal composition of the present invention to provide a load of 50 grams of carbohydrate. Blood glucose measurements, utilizing a Bayer Glucometer and Elite test strips were performed at various time points before and after consumption of the test meal composition. The glucose measurements in mmol/L are shown in Figure 1. The measurements were taken after consuming the test meal on four different occasions following an overnight fast.

### Example 3. Comparison of the Glycemic Responses to the Test Meal and to the Oral Glucose Tolerance Test

Three groups of adults (≥ 18 years of age) were studied: 11 nonobese (body mass index ("BMI"), BMI <27 kg/m²) normal subjects; 5 subjects with impaired glucose tolerance ("IGT") within the last twelve months; and 13 subjects with non-insulin dependent (type 2) diabetes treated by diet alone. The subjects were studied after 10-12 hour overnight fasts on two separate mornings over a two-week period.

Subjects consumed either 75 grams of glucose in 300 ml orange-flavored water (Glucodexe^{®}) or a 100-gram dry carbohydrate diagnostic test meal. The order of tests was randomized, with half the subjects consuming the oral glucose meal test first, and half the test meal first.

The glucose solution was taken with 250 ml water, and test meal was taken with 450 ml water. Both tests were consumed within 10 minutes.

Both venous and capillary blood samples were obtained at each time point. The capillary blood sample was taken from a finger prick immediately after the venous sample had been obtained by way of an intravenous cannula. Blood samples were collected prior to (t₀ = start of test meal consumption) and 30, 60, 90, and 120 minutes after starting to eat and whole blood glucose was analyzed by a clinical laboratory using standard procedures.

Figures 2, 3 and 4 show the kinetics of the blood glucose responses to the test meal and the oral glucose tolerance test for each of the groups studied. The mean fasting blood glucose concentration was similar before each of the two tests. After glucose ingestion, mean blood glucose was significantly greater than after ingestion of the test meal at every time point. However, the glycemic responses for both tests show clear demarcation between the groups allowing differentiation of glycemic status.

### Example 4 Comparison of the Glycemic Responses to the Test Meal from WO 97/02050 and the Present Invention Diabetes Test Meal

A healthy normal individual (> 18 years of age; BMI <27 kg/m²) was studied. After 10-12 hour overnight fasts on two separate mornings over a three-day period.

Subject consumed a test meal as described in either WO 97/02050 or the present invention. The meals were taken with 450 ml water. Both tests were consumed within 10 minutes.

Capillary blood samples were obtained at each time point. The capillary blood sample was taken from a finger prick immediately after the venous sample had been obtained by way of an intravenous cannula. Blood samples were collected prior to (to = start of test meal consumption) and 30, 60, 90, and 120 minutes.

Figure 5 shows the kinetics of the blood glucose responses to each test meal. The mean fasting blood glucose concentration was similar before each of the two tests. After the current test meal ingestion, mean blood glucose was significantly greater than after ingestion of the WO 97/02050 test meal.

The present invention has been described with regard to preferred embodiments.

In the specification the word "comprising" is used as an open-ended term, substantially equivalent to the phrase "including but not limited to", and the word "comprises" has a corresponding meaning. Citation of references is not an admission that such references are prior art to the present invention.

## Claims

1. A solid oral diagnostic test meal, comprising:
(a) from 35 to 55 percent by weight of a glycemic polysaccharide;
(b) from 10 to 35 percent by weight mono- and disaccharides, the mono- and di-saccharides comprising glucose;
(c) from 10 to 25 percent by weight dietary fat, and
(d) from 5 to 8 percent by weight dietary protein,
wherein the solid oral diagnostic test meal comprises less than 0.5 percent by weight soluble fibre, and
wherein the solid oral diagnostic test meal is in a dry form.

2. The solid oral diagnostic test meal according to claim 1, wherein the oral diagnostic test meal comprises less than 0.2 percent by weight soluble fibre.

3. The solid oral diagnostic test meal according to claim 1 or 2, wherein the oral diagnostic test meal comprises from 50 to 60 percent by weight of glycemic carbohydrate.

4. The solid oral diagnostic test meal according to claim 1, 2 or 3, wherein the ratio of (a) to (b) is from 1:1 to 2.8:1.

5. The solid oral diagnostic test meal of claim 1, 2 or 3, wherein the ratio of (a) to (b) is from 1.3:1 to 2.8:1.

6. The solid oral diagnostic test meal of claim 1, 2 or 3, wherein the ratio of (a) to (b) is from 1.4:1 to 2.5:1.

7. The solid oral diagnostic test meal of any one of claims 1 to 6, wherein the monosaccharide is fructose, glucose, or a mixture of glucose and fructose.

8. The solid oral diagnostic test meal of any one of claims 1 to 7, wherein the disaccharide is sucrose.

9. The solid oral diagnostic test meal of any one of claims 1 to 8, wherein said dietary fat comprises from 10 percent to 30 percent saturated fat, and 25 percent to 75 percent monounsaturated fat.

10. The solid oral diagnostic test meal of any one of claims 1 to 9, wherein the glycemic polysaccharide is derived from whole oat flour, defatted oat flour, oat starch, or a mixture thereof.

11. The solid oral diagnostic test meal of any one of claims 1 to 10, further comprising one or both of a source of insoluble dietary fibre, and a source of flavoring.

12. The solid oral diagnostic test meal of any one of claims 1 to 11, wherein the test meal is provided in the form of a bar or biscuit.

13. The solid oral diagnostic test meal of any one of claims 1 to 12, wherein the glycemic polysaccharide is derived from a grain crop selected from the group consisting of barley, oat, wheat, rye, corn, maize, sorghum, millet, rice, amaranth and quinoa.

14. The solid oral diagnostic test meal according to any one of claims 1-13, wherein the solid oral diagnostic test meal comprises a standardized amount of glycemic carbohydrate.

15. The solid oral diagnostic test meal according to any one of claims 1-14 for use in diagnosing a disorder of carbohydrate metabolism in a vertebrate subject.

16. The solid oral diagnostic test according to claim 15, wherein the disorder of carbohydrate metabolism is selected from the group consisting of diabetes mellitus, impaired glucose tolerance, insulin resistance, non-insulin dependent diabetes, maturity onset diabetes, gestational diabetes and hyperinsulinemia.

17. The solid oral diagnostic test meal according to any one of claims 1-14 for use in determining a postprandial glucose concentration in a biological sample from a vertebrate subject.

18. The solid oral diagnostic test meal according to any one of claims 1-14, for use in determining a postprandial insulin response in a vertebrate subject.

19. The solid oral diagnostic test meal according to any one of claims 1-14, for use in diabetes self-diagnosis or self-monitoring in a vertebrate subject..

## Patentansprüche

1. Festes orales diagnostisches Testessen, umfassend:
a) von 35 bis 55 Gew.-% eines glykämischen Polysaccharids,
b) von 10 bis 35 Gew.-% Mono- und Disaccharide, wobei die Mono- und Disaccharide Glucose umfassen,
c) von 10 bis 25 Gew.-% Nahrungsfett und
d) von 5 bis 8 Gew.-% Nahrungsprotein,
wobei das feste orale diagnostische Testessen weniger als 0,5 Gew.-% lösliche Faser umfasst, und
wobei das feste orale diagnostische Testessen in einer trockenen Form vorliegt.

2. Festes orales diagnostisches Testessen nach Anspruch 1, wobei das orale diagnostische Testessen weniger als 0,2 Gew.-% lösliche Faser umfasst.

3. Festes orales diagnostisches Testessen nach Anspruch 1 oder 2, wobei das orale diagnostische Testessen von 50 bis 60 Gew.-% glykämisches Kohlenhydrat umfasst.

4. Festes orales diagnostisches Testessen nach Anspruch 1, 2 oder 3, wobei das Verhältnis von (a) zu (b) von 1:1 bis 2,8:1 beträgt.

5. Festes orales diagnostisches Testessen nach Anspruch 1, 2 oder 3, wobei das Verhältnis von (a) zu (b) von 1,3:1 bis 2,8:1 beträgt.

6. Festes orales diagnostisches Testessen nach Anspruch 1, 2 oder 3, wobei das Verhältnis von (a) zu (b) von 1,4:1 bis 2,5:1 beträgt.

7. Festes orales diagnostisches Testessen nach einem der Ansprüche 1 bis 6, wobei das Monosaccharid Fructose, Glucose oder ein Gemisch von Glucose und Fructose ist.

8. Festes orales diagnostisches Testessen nach einem der Ansprüche 1 bis 7, wobei das Disaccharid Saccharose ist.

9. Festes orales diagnostisches Testessen nach einem der Ansprüche 1 bis 8, wobei das Nahrungsfett von 10% bis 30% gesättigtes Fett umfasst und 25% bis 75% einfach ungesättigtes Fett.

10. Festes orales diagnostisches Testessen nach einem der Ansprüche 1 bis 9, wobei das glylcämische Polysaccharid von Vollhafermehl, entfettetem Hafermehl, Haferstärke oder einem Gemisch davon abgeleitet ist.

11. Festes orales diagnostisches Testessen nach einem der Ansprüche 1 bis 10, welches weiterhin eine Quelle von unlöslicher Nahrungsfaser oder eine Quelle für die Aromatisierung oder beides umfasst.

12. Festes orales diagnostisches Testessen nach einem der Ansprüche 1 bis 11, wobei das Testessen in der Form eines Riegels oder Kleingebäcks bereitgestellt wird.

13. Festes orales diagnostisches Testessen nach einem der Ansprüche 1 bis 12, wobei das glykämische Polysaccharid von einer Getreidenutzpflanze abgeleitet ist, die aus der Gruppe ausgewählt ist, welche aus Gerste, Hafer, Weizen, Roggen, Korn, Mais, Sorghum, Hirse, Reis, Amarant und Quinoa besteht.

14. Festes orales diagnostisches Testessen nach einem der Ansprüche 1 bis 13, wobei das feste orale diagnostische Testessen eine standardisierte Menge an glykämischem Kohlenhydrat umfasst.

15. Festes orales diagnostisches Testessen nach einem der Ansprüche 1 bis 14 für die Verwendung beim Diagnostizieren einer Störung des Kohlenhydratstoffwechsels in einem Wirbeltiersubjekt.

16. Festes orales diagnostisches Testessen nach Anspruch 15, wobei die Störung des Kohlenhydratstoffwechsels ausgewählt ist aus der Gruppe, die aus Diabetes mellitus, Glucosetoleranzstörung, insulinresistenz, nicht-Insulin-abhängige Diabetes, Altersdiabetes, Schwangerschaftsdiabetes und Hyperinsulinämie besteht.

17. Festes orales diagnostisches Testessen nach einem der Ansprüche 1 bis 14 für die Verwendung bei der Bestimmung einer postprandialen Glucosekonzentration in einer biologischen Probe aus einem Wirbeltiersubjekt.

18. Festes orales diagnostisches Testessen nach einem der Ansprüche 1 bis 14 für die Verwendung bei der Bestimmung einer postprandialen Insulinantwort in einem Wirbeltiersubjekt.

19. Festes orales diagnostisches Testessen nach einem der Ansprüche 1 bis 14 für die Verwendung bei der Selbstdiagnose von Diabetes oder der Eigenüberwachung von Diabetes in einem Wirbeltiersubjekt.

## Revendications

1. Repas de test diagnostique à usage oral solide, comprenant :
(a) de 35 à 55 % en poids d'un polysaccharide glycémique ;
(b) de 10 à 35 % en poids de mono- et di-saccharides, les mono- et di-saccharides comprenant du glucose ;
(c) de 10 à 25 % en poids de matières grasses alimentaires, et
(d) de 5 à 8 % en poids de protéines alimentaires,
lequel repas de test diagnostique à usage oral solide comprend moins de 0,5 % en poids de fibres solubles, et
lequel repas de test diagnostique à usage oral solide est sous forme sèche.

2. Repas de test diagnostique à usage oral solide selon la revendication 1, lequel repas de test diagnostique à usage oral comprend moins de 0,2 % en poids de fibres solubles.

3. Repas de test diagnostique à usage oral solide selon la revendication 1 ou 2, lequel repas de test diagnostic à usage oral comprend de 50 à 60 % en poids d'hydrates de carbone glycémiques.

4. Repas de test diagnostique à usage oral solide selon la revendication 1, 2 ou 3, dans lequel le rapport de (a) à (b) est de 1/1 à 2,8/1.

5. Repas de test diagnostique à usage oral solide selon la revendication 1, 2 ou 3, dans lequel le rapport de (a) à (b) est de 1,3/1 à 2,8/1.

6. Repas de test diagnostique à usage oral solide selon la revendication 1, 2 ou 3, dans lequel le rapport de (a) à (b) est de 1,4/1 à 2,5/1.

7. Repas de test diagnostique à usage oral solide selon l'une quelconque des revendications 1 à 6, dans lequel le monosaccharide est le fructose, le glucose, ou un mélange de glucose et de fructose.

8. Repas de test diagnostique à usage oral solide selon l'une quelconque des revendications 1 à 7, dans lequel le disaccharide est le saccharose.

9. Repas de test diagnostique à usage oral solide selon l'une quelconque des revendications 1 à 8, dans lequel lesdites matières grasses alimentaires comprennent de 10 % à 30 % de graisses saturées, et de 25 % à 75 % de graisses monoinsaturées.

10. Repas de test diagnostique à usage oral solide selon l'une quelconque des revendications 1 à 9, dans lequel le polysaccharide glycémique dérive de farine d'avoine complète, de farine d'avoine dégraissée, d'amidon d'avoine, ou d'un de leurs mélanges.

11. Repas de test diagnostique à usage oral solide selon l'une quelconque des revendications 1 à 10, comprenant en outre l'un ou les deux parmi une source de fibres alimentaires insolubles et une source d'agent de saveur.

12. Repas de test diagnostique à usage oral solide selon l'une quelconque des revendications 1 à 11, lequel repas de test est fourni sous la forme d'une barre ou d'un biscuit.

13. Repas de test diagnostique à usage oral solide selon l'une quelconque des revendications 1 à 12, dans lequel le polysaccharide glycémique dérive d'une céréale cultivée choisie dans le groupe constitué par l'orge, l'avoine, le blé, le seigle, le maïs, le maïs doux, le sorgho, le millet, le riz, l'amarante et le quinoa.

14. Repas de test diagnostique à usage oral solide selon l'une quelconque des revendications 1 à 13, lequel repas de test diagnostique à usage oral solide comprend une quantité normalisée d'hydrates de carbone glycémiques.

15. Repas de test diagnostique à usage oral solide selon l'une quelconque des revendications 1 à 14, à utiliser dans le diagnostic d'un trouble du métabolisme des hydrates de carbone chez un sujet vertébré.

16. Repas de test diagnostique à usage oral solide selon la revendication 15, dans lequel le trouble du métabolisme des hydrates de carbone est choisi dans le groupe constitué par le diabète sucré, l'intolérance au glucose, la résistance à l'insuline, le diabète non insulinodépendant, le diabète de type 2, le diabète gestationnel et l'hyperinsulinémie.

17. Repas de test diagnostique à usage oral solide selon l'une quelconque des revendications 1 à 14, à utiliser dans la détermination de la concentration de glucose postprandiale dans un échantillon biologique provenant d'un sujet vertébré.

18. Repas de test diagnostique à usage oral solide selon l'une quelconque des revendications 1 à 14, à utiliser dans la détermination de la réponse insulinique postprandiale chez un sujet vertébré.

19. Repas de test diagnostique à usage oral solide selon l'une quelconque des revendications 1 à 14, à utiliser dans l'autodiagnostic ou l'autosurveillance du diabète chez un sujet vertébré.
